(19) Europäisches Patentamt
European Patent Office
Office européen des brevets

(11) Veröffentlichungsnummer: **0 002 807**
**B1**

(12) # EUROPÄISCHE PATENTSCHRIFT

(21) Anmeldenummer: **78101801.5**

(22) Anmeldetag: **21.12.78**

(51) Int. Cl.[3]: **C 07 D 307/32**

(54) **Verfahren zur Herstellung von Alpha-Hydroxy-Beta,Beta-dimethyl-Gamma-butyrolacton**

(30) Priorität: **30.12.77 DE 2758883**

(43) Veröffentlichungstag der Anmeldung:
**11.07.79 Patentblatt 79/14**

(45) Bekanntmachung des Hinweises auf die Patenterteilung:
**01.10.80 Patentblatt 80/20**

(84) Benannte Vertragsstaaten:
**BE CH DE FR GB IT NL SE**

(56) Entgegenhaltungen:
**DE - A - 2 003 600**
**DE - A - 2 627 940**
**DE - B - 2 228 641**
**US - A - 2 443 334**
**US - A - 2 702 816**
**US - A - 2 852 530**
**US - A - 2 863 878**

(73) Patentinhaber: **BASF Aktiengesellschaft**
**Carl-Bosch-Strasse 38**
**D - 6700 Ludwigshafen (DE)**

(72) Erfinder: **Distler, Harry, Dr.**
**In den Hahndornen 5**
**D - 6719 Bobenheim (DE)**
**Goetze, Walter, Dr.**
**Duerkheimer Strasse 13**
**D - 6701 Dannstadt-Schauernheim 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 002 807 B1

Verfahren zur Herstellung von Alpha-Hydroxy-Beta,Beta-dimethyl-Gamma-butyrolacton

Die Erfindung betrifft ein neues Verfahren zur Herstellung von $\alpha$-Hydroxy-$\beta$,$\beta$-dimethyl-$\gamma$-butyrolacton durch einbadige Umsetzung von Formaldehyd mit Isobutyraldehyd in Gegenwart von tertiären Aminen, nachfolgende Umsetzung mit Blausäure und anschließende Hydrolyse mit Chlorwasserstoffgas unter bestimmten Reaktionsbedingungen bezüglich Temperatur, Reaktionszeit und Blausäurekonzentration.

Es ist aus Ullmanns Encyklopädie der technischen Chemie, Band 18, Seite 205 bekannt, daß man Formaldehyd mit Isobutyraldehyd zum $\alpha$,$\alpha$-Dimethyl-$\beta$-hydroxypropionaldehyd kondensieren kann und diesen Aldehyd dann durch Addition von Blausäure zum Cyanhydrin umwandelt, aus dem durch Verseifung $\alpha$-Hydroxy-$\beta$,$\beta$-dimethyl-$\gamma$-butyrolacton (Pantolacton) entsteht. Nach der US-Patentschrift 2 399 362 wird die Umsetzung so durchgeführt, daß man Formaldehyd und Isobutyraldehyd in Gegenwart von Alkalicyanid umsetzt, das Reaktionsgemisch mit Bisulfit behandelt und das erhaltene Formisobutyraldolcyanhydrin mit starker Mineralsäure hydrolysiert und lactonisiert; im Beispiel wird als Mineralsäure Salzsäure verwendet. Nach der US-Patentschrift 2 852 530 wird für die Kondensation Alkalicarbonat anstelle von Natriumcyanid verwendet, wobei die Temperatur von 25 bis 45°C beträgt. Danach überführt man das Carbonat durch Zusatz von Mineralsäure in das Bicarbonat und setzt das Aldolisierungsgemisch mit einem Mol Alkalibicarbonat und einem Mol Alkalicyanid pro Mol Formisobutyraldol bei einer Temperatur von −5 bis +45°C um. Zur Hydrolyse wird, wie das Beispiel zeigt, Salzsäure verwendet.

Das Verfahren der US-Patentschrift 2 702 816 betrifft die Umsetzung von Glykolsäurenitril mit Isobutyraldehyd und Alkali- oder Erdalkalihydroxiden bei 5 bis 15°C und die Hydrolyse des hierbei entstehenden Cyanhydrins mit Mineralsäure. Die als Nebenprodukte anfallenden Glykole müssen durch Zusatz von Alkalihydroxid zum Hydrolysengemisch und anschließende Wasserdampfdestillation abgetrennt werden. Aus dem Rückstand gewinnt man dann das Verfahrensprodukt durch Behandlung mit Salzsäure, Neutralisation, Filtration, mehrfache Extraktion, Trocknen über wasserfreiem Natriumsulfat, Filtration und Destillation, also auf eine insgesamt umständliche Arbeitsweise.

Nach dem Verfahren der US-Patentschrift 2 863 878 setzt man Isobutyraldehyd und Formaldehyd bei einer Temperatur von 0 bis 30°C in Gegenwart von 0,5 bis 5 Gewichtsprozent eines basischen Katalysators um, gibt Natriumcyanid und dann bei 0 bis 30°C Blausäure hinzu, neutralisiert das Gemisch und läßt es danach mit wäßriger Salzsäure reagieren. Die US-Patentschrift lehrt, daß die Umsetzung in alkoholischer, z.B. methanolischer Lösung (Spalte 1, Zeile 70 bis Spalte 2, Zeile 2) durchgeführt wird. Zwar können auch basische Katalysatoren wie organische Amine, Triäthylamin und quaternäre Amminiumhydroxide verwendet werden, es wird aber ausdrücklich festgestellt, daß Natriumhydroxid bevorzugt wird. Die Beispiele zeigen, daß man mit Isobutyraldehyd und Formaldehyd als Ausgangsstoffen (Beispiel 1) nur Natriumhydroxid als Katalysator verwendet. Beispiel 2 und 3 zeigen, daß lediglich im Falle des schon hergestellten und isolierten Hydroxypivalaldehyd als Ausgangsstoff sowohl Natriumhydroxid als auch Triäthylamin in Frage kommen. Blausäure muß mindestens in äquimolarer Menge in alkoholischer Lösung zugesetzt werden; wie Beispiel 1 und die Beschreibung (Spalte 2, Zeilen 50 bis 55; Spalte 3, Zeile 29) zeigen, wird jedoch im allgemeinen überschüssige Blausäure verwendet. Es wird angegeben und im Beispiel gezeigt, daß die Umsetzung zweckmäßig bei 0 bis 15°C durchgeführt wird. Das Verfahren macht die Beseitigung der überschüssigen Blausäure und der Lösungsmittel notwendig und ist daher technisch aufwendig. Setzt man Natriumcyanid als Reaktionspartner bzw. als Katalysator ein, so braucht man eine Hilfssäure, um Blausäure in der Reaktionsmischung freizusetzen. Am Ende erhält man Neutralsalzlösungen, die Cyanidionen enthalten und deren Beseitigung aus Umweltgründen einen erheblichen technischen Aufwand erforderlich machen. Bei diesem Verfahren werden nur wäßrige verdünnte Mineralsäuren zur Hydrolyse verwendet.

Es wurde nun gefunden, daß man $\alpha$-Hydroxy-$\beta$,$\beta$-dimethyl-$\gamma$-butyrolacton durch Kondensation von Formaldehyd und Isobutyraldehyd zum Hydroxypivalaldehyd, dessen Überführung in sein Cyanhydrin sowie nachfolgende saure Hydrolyse und Lactonisierung vorteilhaft erhält, wenn man in einem ersten Schritt Formaldehyd und Isobutyraldehyd in Gegenwart von 0,01 bis 0,3 Mol eines tertiären Amins je Mol Isobutyraldehyd während 30 bis 50 Minuten bei einer Temperatur von 80 bis 95°C umsetzt, dann in einem zweiten Schritt das so gebildete Reaktionsgemisch mit Blausäure während 60 bis 120 Minuten bei einer Temperatur von 20 bis 45°C reagieren läßt, wobei die Konzentration der Blausäure während der Reaktion nicht mehr als 1 Gewichtsprozent, bezogen auf das Reaktionsgemisch, beträgt, und dann in einem dritten Schritt das so gebildete Reaktionsgemisch mit gasförmigem Chlorwasserstoff umsetzt.

Die Umsetzung verläuft nach folgendem Schema:

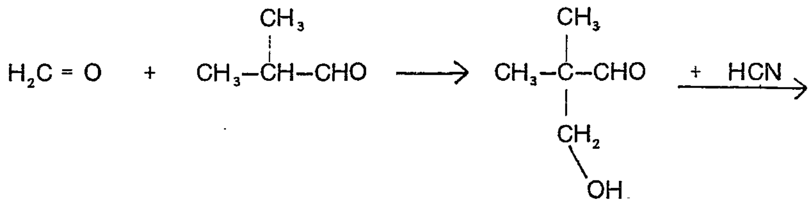


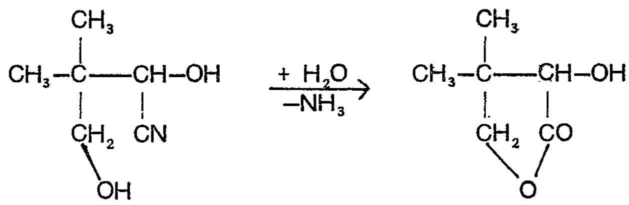


Im Vergleich zu den bekannten Verfahren liefert das Verfahren nach der Erfindung auf einfacherem und wirtschaftlicherem Wege $\alpha$-Hydroxy-$\beta$,$\beta$-dimethyl-$\gamma$-butyrolacton in besserer Ausbeute und Reinheit. Das Verfahren ist besonders gut für den Betrieb im großtechnischen und kontinuierlichen Maßstab geeignet, bietet keine wesentlichen Abwasserfragen und ist umweltfreundlicher als die bisher bekannten Verfahren. Da man in der Regel Blausäure in etwa stöchiometrischer Menge, bezogen auf Isobutyraldehyde, verwendet, sind die so anfallenden Pantolactonlösungen praktisch cyanidfrei. Ein weiterer bemerkenswerter Vorteil des erfindungsgemäßen Verfahrens ist die Tatsache, daß durch die stets nur geringen Überschüsse an Blausäure ein sehr reines, praktisch cyanidfreies D,L-Pantolacton entsteht, das sofort zu Pantothensäure verarbeitet werden kann.

Alle diese Vorteile sind im Hinblick auf den Stand der Technik als überraschend zu bezeichnen.

Formaldehyde kann in flüssiger Form oder als Gas im allgemeinen in Form seiner wäßrigen, zweckmäßigerweise 10- bis 50-, vorzugsweise von 30- bis 40-gewichtsprozentigen Losung verwendet werden. Blausäure wird als Gas oder zweckmäßigerweise in flüssiger Form verwendet. Man kann die Aldehyde in stöchiometrischer Menge oder jede der Komponenten jeweils im Überschuß einsetzen, vorzugsweise in einer Menge von 0,9 bis 1,5, insbesondere 0,9 bis 1,1 Mol Formaldehyd je Mol Isobutyraldehyd.

Die Umsetzung wird im ersten Schritt in Gegenwart eines tertiären Amins in einer Menge von 0,01 bis 0,3, vorzugsweise von 0,06 bis 0,1 Mol des tertiären Amins, bezogen auf ein Mol Isobutyraldehyd, durchgeführt. Geeignete Amine sind z.B. Trimethylamin, Triäthylamin, Tripropylamin, Triisopropylamin, Tributylamin, Triisobutylamin, Tri-sek.-butylamin, Tri-tert.-butylamin, Tribenzylamin, Tricyclohexylamin, Triamylamin, Trihexylamin, N,N-Dimethylanilin, N,N-Diäthylanilin, N,N-Dipropylanilin, N,N-Dimethyltoluidin, N,N-Diäthyltoluidin, N,N-Dipropyltoluidin, N,N-Dimethyl-p-aminopyridin, N,N-Diäthyl-p-aminopyridin, N,N-Dipropyl-p-aminopyridin, N,N-Dimethylaminoäthanol, N,N-Diäthylaminoäthanol, N,N-Dipropylaminoäthanol, N-Methylpiperidin, N-Äthylpiperidin, N-Methylpyrrolidin, N-Äthylpyrrolidin, N-Methylimidazol, N-Äthylimidazol, N-Methylpyrrol, N-Äthylpyrrol, N-Methylmorpholin, N-Äthylmorpholin, N-Methylhexamethylenimin, N-Äthylhexamethylenimin, Pyridin, Chinolin, $\alpha$-Picolin, $\beta$-Picolin, $\gamma$-Picolin, Isochinolin, Pyrimidin, Acridin, N,N,N',N'-Tetramethyläthylendiamin, N,N,N',N'-Tetraäthyläthylendiamin, Chinoxalin, Chinazolin, N-Propyldiisopropylamin, N,N-Dimethylcyclohexylamin, 2,6-Lutidin, 2,4-Lutidin, Trifurfurylamin und Triäthylendiamin; besonders vorteilhaft sind die genannten Trialkylamine mit 1 bis 4 Kohlenstoffatomen je Alkylgruppe.

Im ersten Schritt wird die Umsetzung bei 80 bis 95, vorzugsweise von 84 bis 91°C, bei Unterdruck oder Überdruck oder vorzugsweise bei Normaldruck kontinuierlich durchgeführt. Die Reaktionszeit beträgt 30 bis 50, vorzugsweise 35 bis 45 Minuten. In einer bevorzugten Ausführungsform teilt man den ersten Schritt in zwei Unterschritte, z.B. mittels zweier nachgeschalteter Reaktoren, mit zweckmäßig zuerst a) einer Temperatur von 80 bis 90, insbesondere 83 bis 88°C und einer Verweilzeit von 15 bis 25 Minuten, insbesondere 18 bis 22 Minuten und dann b) einer Temperatur von 85 bis 95°C, insbesondere 88 bis 93°C, und einer Verweilzeit von 15 bis 25 Minuten, insbesondere 18 bis 22 Minuten.

Blausäure wird dem Ausgangsgemisch im zweiten Schritt in einer solchen Menge zugegeben, daß die Konzentration während der Reaktion nicht mehr als 1, zweckmäßig von 0,01 bis 1, vorzugsweise nicht mehr als 0,1, vorzugsweise von 0,01 bis 0,1, insbesondere von 0,01 bis 0,06 Gewichtsprozent Blausäure, bezogen auf das Reaktionsgemisch, beträgt. Bevorzugt verwendet man Wasser beim ersten und zweiten Schritt allein als Lösungsmittel, gegebenenfalls auch unter den Reaktionsbedingungen inerte, organische Lösungsmittel. Die organischen Lösungsmittel sind prinzipiell nicht notwendig, können sich aber aus verfahrenstechnischen Gründen, z.B. wegen besserer Dosierung oder einer erleichterten Temperaturführung, empfehlen.

Zweckmäßig verwendet man Wasser in einer Menge von 5 bis 50 Gewichtsprozent, vorzugsweise von 10 bis 20 Gewichtsprozent, bezogen auf Isobutyraldehyd. Bevorzugt gelangt Wasser nur in Form

der wäßrigen Formaldehydlösung in die Gesamtreaktion. Man kann Blausäure und die Aldehyde in etwa stöchiometrischer Menge oder jede der Komponenten jeweils im Überschuß einsetzen, vorzugsweise in einer Menge von 0,9 bis 1,1 Mol, insbesondere von 0,99 bis 1,01 Mol Blausäure pro Mol Isobutyraldehyd, vor allem jedoch in stöchiometrischer Menge. Man kann die Blausäure vorlegen und das Gemisch des ersten Schrittes dazu geben oder vorteilhaft die Blausäure in das Gemisch selbst einleiten. Der zweite Schritt kann unter Überdruck, Unterdruck oder zweckmäßigerweise Normaldruck durchgeführt werden. Die Reaktionszeit im zweiten Schritt beträgt 60 bis 120, vorzugsweise 90 bis 110 Minuten und die Reaktionstemperatur 20 bis 45, vorzugsweise 20 bis 30°C. In einer bevorzugten Ausführungsform teilt man den zweiten Schritt in zwei Unterschritte, z.B. mittels zweier nachgeschalteter Reaktoren, mit zweckmäßig zuerst a) einer Verweilzeit von 20 bis 70, vorzugsweise 40 bis 60 Minuten und dann einer Verweilzeit b) von 20 bis 70, vorzugsweise 40 bis 60 Minuten bei den vorgenannten Temperaturen.

Ein besonderes Merkmal des erfindungsgemäßen Verfahrens ist im dritten Reaktionsschritt die Verwendung von gasförmigem Chlorwasserstoff als Hydrolysierungs- und Lactonisierungsmittel. Dieser Schritt wird zweckmäßig bei 50 bis 150, insbesondere 75 bis 120°C, unter erhöhtem Druck oder unter Normaldruck, kontinuierlich und mit Mengen von 1,05 bis 2, vorzugsweise 1,05 bis 1,25 Mol HCl je Mol des Cyanhydrins, durchgeführt. Die Verweilzeit beträgt vorteilhaft 10 bis 300, vorzugsweise 30 bis 260 Minunten. In einer bevorzugten Verfahrensweise teilt man den dritten Schritt in zwei Unterschritte, z.B. mittels zweier nachgeschalteter Reaktoren, mit zweckmäßig zuerst a) Temperaturen von 70 bis 100°C, vorzugsweise 75 bis 95°C und Verweilzeiten von 1 bis 2,5 Stunden, vorzugsweise 1,8 bis 2,2 Stunden, und dann b) Temperaturen von 90 bis 120, vorzugsweise 100 bis 115°C und Verweilzeiten von 1 bis 2,5 Stunden, vorzugsweise 1,8 bis 2,2 Stunden.

Alle drei Schritte können wie folgt durchgeführt werden: Ein gemisch aus Formaldehyd, Wasser, Isobutyraldehyd und dem tertiären Amin wird während der Reaktionszeit bei den Reaktionstemperaturen gehalten. Man gibt im zweiten Schritt die Blausäure ins Ausgangsgemisch, und zwar entweder in Portionen oder, vorteilhafter, kontinuierlich so zu, daß die vorgenannte Blausäurekonzentration während der gesamten Reaktionszeit des zweiten Schrittes eingehalten wird. Die laufende Messung der Blausäurekonzentrationen kann z.B. durch Titration nach Vollhard (Ullmann, loc. cit., Band 5, Seite 666) oder zweckmäßigerweise über eine Silber-Kalomelelektrode erfolgen. Im dritten Schritt wird dem Gemisch Chlorwasserstoffgas zugefügt. Dann wird das Pantolacton aus dem Reaktionsgemisch in üblicher Weise, beispielsweise durch Extraktion mit Methylenchlorid und Destillation des Lösungsmittels, isoliert. Im allgemeinen wird man aber schon aus betrieblichen und wirtschaftlichen Gründen die nach dem dritten Schritt angefallene Lösung des Pantolactons direkt weiterverarbeiten, z.B. zu Pantothensäure, was gerade bei dem erfindungsgemäßen Verfahren infolge der Reinheit dieser Lösungen vorteilhaft ist.

Das nach dem Verfahren der Erfindung erhältliche Pantolacton ist ein wertvoller Ausgangsstoff für die Herstellung von Pharmazeutika und Vitaminen, insbesondere von Pantothensäure. Bezüglich der Verwendung wird auf die vorgenannten Veröffentlichungen und Ullmans Encyklopädie der technischen Chemie, Band 18, Seiten 201 bis 203, verwiesen.

Die in dem folgenden Beispiel aufgeführten Teile sind Gewichsteile.

Beispiel

Durch einen Reaktor, der mit Rührer, Rückflußkühler und 3 Zulaufgefäßen ausgestattet ist, wurden stündlich bei 85°C 670 Teile 30-gewichtsprozentiger wäßriger Formaldehyd, 484 Teile Isobutyraldehyd und 46,5 Teile Triäthylamin geleitet. Die Verweilzeit betrug 20 Minuten. In einem folgenden Reaktor wurde das Reaktionsgemisch 20 Minuten bei 90°C gehalten. In einem nachfolgenden Reaktor wurden dem Reaktionsgemisch stündlich 181 Teile Blausäure bei 25°C zugegeben, wobei im Reaktionsraum die Blausäurekonzentration von 0,1 Gew.% nicht überschritten wurde (Mittelwert 0,01 bis 0,05 Gew.% HCN). Die Verweilzeit betrug 50 Minuten. In einem Nachreaktor mit einer Verweilzeit von 50 Minuten (25°C) fiel die Blausäurekonzentration des kontinuierlich durchgeleiteten Reaktionsgemisches auf unter 4 ppm ab. In einem nachgeschalteten Rührbehälter mit Rückflußkühler und Gaszuführung wurde das Reaktionsgemisch bei 85°C stündlich mit 317 Teilen gasförmigem HCl umgesetzt. Nach einer Verweilzeit von 2 Stunden floß das Reaktionsgemisch in einen Nachreaktor, wo es 2 Stunden bei 110°C gehalten wurde. Durch Extraktion mit stündlich 700 Teilen Methylenchlorid und fraktionierte Destillation des Extrakts wurden stündlich 836 Teile Pantolacton (96% der Theorie) vom Fp (Methylenchlorid) 88°C erhalten.

**Patentanspruch**

Verfahren zur Herstellung von $\alpha$-Hydroxy-$\beta$,$\beta$-dimethyl-$\gamma$-butyrolacton durch Kondensation von Formaldehyd und Isobutyraldehyd, Überführung zum Hydroxypivalaldehyd, dessen Überführung in sein Cyanhydrin sowie nachfolgende saure Hydrolyse und Lactonisierung, *dadurch gekennzeichnet,* daß man in einem ersten Schritt Formaldehyd und Isobutyraldehyd in Gegenwart von 0,01 bis 0,3 Mol eines tertiären Amins je Mol Isobutyraldehyd während 30 bis 50 Minuten bei einer Temperatur von 80 bis 95°C umsetzt, dann in einem zweiten Schritt das so gebildete Reaktionsgemisch mit Blausäure

während 60 bis 120 Minuten bei einer Temperatur von 20 bis 45°C reagieren läßt, wobei die Konzentration der Blausäure während der Reaktion nicht mehr als 1 Gewichtsprozent, bezogen auf das Reaktionsgemisch, beträgt, und dann in einem dritten Schritt das so gebildete Reaktionsgemisch mit gasförmigem Chlorwasserstoff umsetzt.

**Claim**

A process for the preparation of $\alpha$-hydroxy-$\beta$,$\beta$-dimethyl-$\gamma$-butyrolactone by condensation of formaldehyde and isobutyraldehyde, conversion to hydroxypivalaldehyde, conversion of the latter to its cyanohydrin, and subsequent acid hydrolysis and lactonization, *characterized in that,* in a first step, formaldehyde and isobutyraldehyde are reacted in the presence of from 0.01 to 0.3 mole of a tertiary amine per mole of isobutyraldehyde for from 30 to 50 minutes at from 80 to 95°C thereafter, in a second step, the reaction mixture thus formed is reacted with hydrocyanic acid for from 60 to 120 minutes at from 20 to 45°C, with the concentration of the hydrocyanic acid not exceeding 1 percent by weight, based on the reaction mixture, during the reaction, and thereafter, in an third step, the resulting mixture is reacted with gaseous hydrogen chloride.

**Revendication**

Procédé pour la préparation d'$\alpha$-hydroxy-$\beta$,$\beta$-diméthyl-$\gamma$-butyrolactone par condensation de formaldéhyde et d'isobutyraldéhyde, transformation en hydroxypivalaldéhyde, conversion de celui-ci en sa cyanhydrine, suivie d'hydrolyse acide et de lactonisation, caractérisé en ce qu'en un premier temps, on fait réagir le formaldéhyde et l'isobutyraldéhyde en présence de 0,01 à 0,3 mole d'une amine tertiaire par mole d'isobutyraldéhyde, pendant 30 à 50 mm à une température de 80 à 95°C, en ce qu'en un second temps, on fait réagir le mélange réactionnel ainsi formé avec l'acide cyanhydrique pendant 60 à 120 mn à une température de 20 à 45°C, la concentration de l'acide cyanhydrique pendant la réaction ne dépassant pas 1% en poids par rapport au melange réactionnel, et en ce qu'en un troisième temps, on fait réagir le mélange réactionnel ainsi formé avec de l'acide chlorhydrique gazeux.